# EUROPEAN PATENT APPLICATION

(11) **EP 4 292 696 A1**
(43) Date of publication of application: **20.12.2023**
(21) Application number: 22179589.1
(22) Date of filing: 17.06.2022
(51) Int. Cl.: B01D 71/10, B01D 15/12, B01D 15/38, B01D 71/16, C07K 1/34

(54) **FILTER SYSTEM USABLE AS CELL CULTURE CLARIFICATION FILTER IN PROTEIN PURIFICATION**

(71) Applicant: Sartorius Stedim Biotech GmbH, 37079 Göttingen (DE)
(72) Inventor: GENCOGLU, Ceren, 37081 Göttingen (DE); LEUTHOLD, Martin, 37079 Göttingen (DE); BAPTISTA, Prisca, 13600 La Ciotat (FR)
(74) Representative: Müller-Boré & Partner Patentanwälte PartG mbB

(57) **Abstract**

The present invention relates to a filter system, which can be used as a clarification filter/depth filter for cell culture clarification before chromatography and/or ultrafiltration for protein purification, as well as to a method of separating cells and other contaminants from a fluid containing one or more target components by employing the filter system of the present invention.

## Description

The present invention relates to a filter system, which can be used as a clarification filter/depth filter for cell culture clarification before chromatography and/or ultrafiltration for protein purification, as well as to a method of separating cells and other contaminants from a fluid containing one or more target components by employing the filter system of the present invention.

The production of recombinant proteins, in cell-based and non-cell-based systems, usually requires the more or less laborious purification of the protein of interest out of a complex mixture. Relevant contaminants in this respect include host cell-derived contaminants, such as whole cells, cell debris, host cell nucleic acids and host cell proteins, culture medium-derived contaminants, such as proteinaceous and non-proteinaceous medium components, and vector-derived contaminants, including vector nucleic acids and viral vectors.

The clarification of cell culture harvests and high-solids feedstocks can be a daunting task due to the large volumes of harvest from modern production batch bioreactors and high cell densities that often require primary clarification prior to the subsequent chromatography operations. As product molecule titers have increased, the higher cell mass and larger amounts of product create challenges for the downstream purification steps. Higher cell densities result in difficulties during clarification and sterile filtration. Higher product concentrations generally result in increased impurity load and the need for larger chromatography installations. As such, improvements in the form of gains in efficiency and throughput are greatly sought after.

Most of the cell components are removed from the liquid by filtration using a depth filter/clarification filter before chromatography and/or ultrafiltration. However, a surprisingly large amount, especially of small or soluble constituents, remain in the liquid as it is applied to the purification system. Many of these compete with the desired protein and bind to the affinity material in the column or clog the ultrafilter.

Current clarification filters used for cell clarification in market are cellulose-based depth filters which need pre-venting and high amounts of flushing prior to filtration because they consist of natural components like diatomaceous earth. Such depth filter materials release high amounts of TOC (Total Organic Carbon) and particles. Furthermore, the batch-to-batch consistency is low for natural-based materials. Such filters have larger void volumes due to larger surface area needed for larger feed volumes which causes high product loss and high unspecific binding. Thus, large volumes of post-flushing are needed to be able to recover the target product from the depth filter. Since conventional depth filter materials are absorptive, lots of particles but also product itself is retained. When large volumes of post-flushing are needed, the final concentration of the product decreases due to dilution. That might cause issues to be able to use the product for further analytical measurements or the process will need additional concentration steps afterwards.

In view of the above, the technical problem underlying the present invention is to provide a filter system usable as a clarification filter/depth filter for cell culture clarification before chromatography and/or ultrafiltration, the filter system exhibiting high filterability (up to 40 million cells/mL), low amount of TOC (Total Organic Carbon) release, high throughput, high yield of a target component(s) and achieving a high reduction of turbidity of a fluid to be clarified.

The solution to the above technical problem is achieved by the embodiments characterized in the claims.

In particular, the present invention provides a filter system comprising, in this order,
a first synthetic non-woven layer having a first average pore size A;
a second synthetic non-woven layer having a second average pore size B;
a third synthetic non-woven layer having a third average pore size C;
a fourth synthetic non-woven layer having a fourth average pore size D; and
a membrane layer having a fifth average pore size E,
wherein
the first average pore size A is from 15.0 µm to 50.0 µm,
the second average pore size B is from 7.50 µm to 30.0 µm,
the third average pore size C is from 5.00 µm to 15.0 µm,
the fourth average pore size D is from 3.00 µm to 10.0 µm,
the fifth average pore size E is from 0.010 µm to 2.5 µm, and
A>B>C>D>E.

The filter system of the present invention is designed to be used prior to the capture step during protein purification and removes large particles and protects a downstream membrane adsorber from fouling and clogging. Due to the use of synthetic non-woven layers in the filter system, the release of high amounts of TOC can be inhibited when compared to conventional depth filters based on natural components such as diatomaceous earth. Thus, the filter system of the present invention can be preferably used as a prefilter system/clarification filter system, particularly a cell culture clarification filter system.

According to the present invention the filter system comprises, in this order from the upstream side to the downstream side thereof, four synthetic non-woven layers and a (final) membrane layer.

According to the invention, a "layer" is understood to mean a planar structure. "Planar" means that the structure has two opposite main surfaces which are predominantly level and are largely parallel to one another, and having a defined thickness between said opposite main surfaces. "Main surfaces" are the faces of the structure having the greatest surface area.

The synthetic non-woven layers differ in average pore size and have been aligned so that the average pore size decreases from the top (upstream) layer (i.e. the first synthetic non-woven layer) to the bottom (downstream) layer (i.e. the fourth synthetic non-woven layer). That is, the first synthetic non-woven layer has a first average pore size A, the second synthetic non-woven layer has a second average pore size B, the third synthetic non-woven layer has a third average pore size C, and the fourth synthetic non-woven layer has a fourth average pore size D, wherein A > B > C > D. This constitution achieves an excellent sieving effect and retention.

According to the present invention, the average pore sizes of the synthetic non-woven layers are determined according to ASTM 1294-89 (1999) using Topas PSM 165 (porometer).

According to the present invention, the average pore sizes of the membrane layer(s) are determined in accordance with the following methods:
- For average pore sizes of at least 0.1 µm to at most 10 µm, capillary flux porometry is used to determine the average pore size of the membrane layer(s). This is a gas/liquid porosimetry in which the differential gas pressures and flow rates through a membrane sample are measured first in the wet and then in the dry state. Prior to measurement, the membrane sample is brought into contact with a wetting liquid in such a way that all pores are filled with this liquid. After the pores have been filled and the sample has been introduced, the measuring cell must be closed and the measurement started. After starting the measurement, the gas pressure is automatically and gradually increased and the pore diameters corresponding to the applied pressure are emptied by the gas pressure. This is continued until the relevant pore range has been covered, i.e. until even the smallest pores present in the measuring range have been freed of liquid. The pressure is then reduced again and the measurement is repeated automatically on the now dry sample. The pore size distribution is calculated from the difference between the two pressure-flow rate curves using the Young-Laplace equation (see also A. Shrestha, "Characterization of porous membranes via porometry", 2012, Mechanical Engineering Graduate Theses & Dissertations, Paper 38, University of Colorado at Boulder).
- For pore sizes less than 0.1 µm, the liquid-liquid displacement method, which has similarities to capillary flow porometry, is used. However, instead of the gas flow rates, the flow rates of the displacing liquid are measured as a function of the differential pressure increase (see also R. Dávila, "Characterization of ultra and nanofiltration commercial filters by liquid-liquid displacement porosimetry", 2013).

According to the present invention, the first average pore size A is from 15.0 µm to 50.0 µm (preferably from 20.0 to 30.0 µm), the second average pore size B is from 7.50 µm to 30.0 µm (preferably from 15.0 to 25.0 µm), the third average pore size C is from 5.00 µm to 15.0 µm (preferably from 7.50 to 12.5 µm), and the fourth average pore size D is from 3.00 µm to 10.0 µm (preferably from 5.00 to 7.50 µm) and A > B > C > D. In case any of the synthetic non-woven layers has an average pore size below the respective lower limit, the throughput of the filter system may be insufficient, and the respective synthetic non-woven layer having an average pore size below the respective lower limit is blocked fast due to overload of the particles. In case any of the synthetic non-woven layers has an average pore size above the respective upper limit, a high reduction of turbidity of the fluid to be clarified may not be achieved. Moreover, a synthetic non-woven layer which has an average pore size above its respective upper limit lets rather big particles pass so that the following synthetic non-woven layer is blocked early. Thus, the filtrate volume/m², i.e. the capacity of the filter system decreases.

The material used for each of the first, second, third and fourth synthetic non-woven layers is not particularly limited. However, according to a preferred embodiment of the present invention, glass fibers are not used as a material for the synthetic non-woven layers due to a potential high release amount of TOC and fiber fragments and large volumes of (post)-flushing needed.

Preferably, the material used for each of the first, second, third and fourth synthetic non-woven layers is selected from the group consisting of polyolefins (such as e.g. polyethylene, polypropylene, polybutylene, etc.), fluoropolyolefins (such as e.g. PTFE), polyamides (such as e.g. Nylon) and polyesters. According to a particularly preferred embodiment, the synthetic non-woven layers are each composed of (meltblown) polypropylene.

According to a preferred embodiment, the first, second, third and fourth synthetic non-woven layers each have an average thickness of from 20 to 20,000 µm, preferably from 50 µm to 10,000 µm, from 60 µm to 5,000 µm, from 70 µm to 2,000 µm, from 80 µm to 1,000 µm, from 90 µm to 500 µm, and most preferably from 100 µm to 300 µm. In case any of the synthetic non-woven layers has a thickness above the respective upper limit, an inner room of the housing design for the filter system may be needed, which may result in a larger void volume of the filter system. A low void volume is advantageous since trapping of any product volume within the filter system housing can be reduced. In case any of the synthetic non-woven layers has a thickness below the respective lower limit, the filter system of the present invention may have less capacity for the particles to be removed from the feed solution. The average thickness of each of the first, second, third and fourth synthetic non-woven layers may be measured according to DIN EN ISO 12625-3 using Universal thickness gauge S16502 (Frank-PTI).

Synthetic non-woven layers having the features as described above are commercially available.

The (final) membrane layer of the filter system of the present invention is not particularly limited as long as the average pore size E thereof is from 0.010 µm to 2.5 µm, as determined according to the methods described hereinabove. The membrane layer has a first main surface and a second main surface opposite the first main surface, and the first main surface is arranged (preferably directly) adjacent to the fourth synthetic non-woven layer. The membrane layer may be a single membrane layer or may be a combination of two or more (separate) membrane layers. In case the membrane layer is a combination of two or more membrane layers, each of the two or more membrane layers has an average pore size E of from 0.010 µm to 2.5 µm.

Depending on the intended application of the filter system of the present invention, either a hydrophilic or a hydrophobic membrane may be used. A membrane layer is considered to be a hydrophilic membrane in case its water contact angle is 90° or less, whereas a hydrophobic membrane has a water contact angle of more than 90°. In case the filter system of the present invention is used as a clarification filter/depth filter for cell culture clarification before chromatography and/or ultrafiltration for protein purification, the membrane layer preferably is a hydrophilic membrane layer, i.e. having a water contact angle of 90° or less, preferably of 80° or less, more preferably of 70° or less. The water contact angle may be determined according to "Standard Test Method for Measurement of the Surface Tension of Solid Coatings, Substrates and Pigments using Contact Angle Measurements" as defined in ASTM D7490-13.

According to a preferred embodiment of the present invention, the average pore size at the first main surface of the membrane layer (which is adjacent to the fourth synthetic non-woven layer) is larger than the average pore size at the (opposite) second main surface. Such configuration of the membrane layer allows a high throughput and achieves a high reduction of turbidity of a fluid to be clarified.

According to a particularly preferred membrane, the membrane layer is an asymmetrical membrane layer. As used herein, an asymmetrical membrane layer is a membrane layer, in which the first main surface of the asymmetrical membrane layer (which is adjacent to the fourth synthetic non-woven layer) has an average pore size larger than the average pore size of the second main surface, and the average pore size (continuously or discontinuously) decreases from the first main surface towards the second main surface of the asymmetrical membrane layer. If the average pore size of a section downstream in the filtration direction is greater than the average pore size of the upstream section, the region of the downstream section having a greater pore size remains substantially unused because the medium has already flowed through a region having a smaller pore size. An asymmetrical membrane layer can advantageously avoid such a so-called "wastage" or a "dead volume", which is only available for soil absorption (absorption of components to be filtered) with limited efficiency.

As described above, the membrane layer may be a single membrane layer or a combination of two or more membrane layers. However, a single membrane layer is preferred due to the simpler manufacturing process thereof and the typically higher possible flow rate when compared to multiple membrane layers.

The material used for the membrane layer is not particularly limited. Accordingly, the membrane layer may be composed of one or more polymers selected from the group consisting of cellulose and derivatives thereof (such as cellulose hydrate, cellulose esters, cellulose nitrate, cellulose acetate), regenerated cellulose, polyamides (such as nylon and perlon), fluoropolymers (such as polytetrafluoroethylene (PTFE) and polyvinylidene difluoride (PVDF), polyolefins (such as polyethylene and polypropylene), polysulfones (such as polysulfone, polyarylsulfone and polyethersulfone), poly(meth)acrylic acid, and acrylic acid/methacrylic acid copolymers. According to a particularly preferred embodiment, the membrane layer is composed of regenerated cellulose or cellulose acetate due to their specific hydrophilicity.

The average pore size E of the membrane layer is from 0.010 µm to 2.5 µm, as determined according to the method described hereinabove. In case the membrane layer is a combination of two or more membrane layers, each of the two or more membrane layers has an average pore size E of from 0.010 µm to 2.5 µm. Due to the specific average pore size E, the filter system of the present invention achieves both high throughput and high reduction of turbidity of a fluid to be clarified. The average pore size E of the membrane layer preferably is from 0.050 µm to 2.0 µm, more preferably from 0.10 µm to 1.5 µm, more preferably from 0.15 µm to 1.0 µm, and most preferably from 0.20 µm to 0.70 µm.

According to a preferred embodiment, the membrane layer has an average thickness of from 20 to 400 µm, preferably from 40 µm to 350 µm, from 60 µm to 300 µm, from 80 µm to 250 µm, from 100 µm to 200 µm, from 120 µm to 180 µm, and most preferably from 140 µm to 170 µm. If the average thickness is below the lower limit, the filter system of the present invention may exhibit a lower throughout and early blocking. If the average thickness is above the upper limit, the filter system of the present invention may only allow a reduced flow rate. If the membrane layer is a combination of two or more membrane layers, the above average thickness ranges refer to the total average thickness of the two or more membrane layers.

Membrane layers having the features as described above are commercially available.

The shape (spatial form) of each of the first, second, third and fourth synthetical non-woven layer and of the membrane layer not subject to any particular restriction. Preferably, each of the filter layers is a flat filter layer. The term "flat" indicates that the respective filter material lies substantially in a single plane having a specific thickness. Preferably, all filter materials lie more or less in planes that are substantially parallel to each other.

The type of connection between each of the first, second, third and fourth synthetic non-woven layers and the membrane layer is not particularly limited. For example, the layers may be loosely connected within a housing or the layers may be sealed by clamping (at the edges thereof), heat sealing overmolding (plastic silicone), etc. According to a preferred embodiment, the layers are loosely connected (within a housing, i.e. the connection between the layers id driven by the connection to the housing) without lamination therebetween.

The filter system of the present invention may consist of the first, second, third and fourth synthetic non-woven layers and the membrane layer. Alternatively, the filter systems of the present invention may comprise additional layers such as e.g. supporting layers which do not impair the effects of the present invention.

The present invention further provides a method of separating cells and other contaminants from a fluid containing one or more target components, the method comprising
providing the filter system of the present invention, wherein the first synthetic non-woven layer is defined as the upstream side of the filter system and the membrane layer is defined as the downstream side of the filter system, and
passing the fluid through the filter system from the upstream side to the downstream side of the filter system to retain the cells and other contaminants in the filter system while eluting the fluid containing the one or more target components from the filter system.

The target component is not specifically limited. For example, the target component may be any biomolecule of interest, such as proteins, antibodies, hormones, vaccines, nucleic acids, exosomes, and viruses, and virus-like particles. In a preferred embodiment, the target component is an antibody, more preferably a monoclonal antibody (mAb), or fragments or derivatives thereof, or nanobodies. Examples of monoclonal antibodies are adalimumab, cetuximab, rituximab, infliximab, omalizumab, and denosumab. The target component can be obtained, for example, from mammalian cells, bacteria cells or insect cells, media and cell lines, such as "Chinese hamster ovary cells" (CHO cells), HeLa, or human umbilical vein endothelial cells (HUVEC).

The source of the fluid is not specifically limited. For example, the fluid can be obtained by applying any biological, biochemical, chemical, or pharmaceutical method. Thereby, the fluid can be obtained by previously conducting other purification methods applying different purification units. For example, the target component can be produced by appropriate cell lines, such as CHO cell lines e.g. by perfusion cultivation.

The fluid is not particularly limited as long as it contains a plurality of components, wherein at least one component of the plurality of components of the fluid is the target component. The further (impurity) components (contaminants) are not particularly limited and may depend on the preparation conditions of the target component. Examples of further components are aggregates, host cell proteins, deoxyribonucleic acid, as well as fragments and charge variants thereof.

The medium of the fluid is not particularly limited. In principle, any fluid is suitable, with water and aqueous salt solutions being preferred. For example, an aqueous buffer solution can be used as the medium of the fluid. Preferably, the medium of the fluid is selected from the group consisting of KPI buffer, sodium phosphate buffer, sodium acetate buffer, PBS, glycine, citrate buffer, Tris buffer, BIS-Tris buffer, HEPES buffer, and water. The concentration of the buffer in the aqueous solution is not particularly limited and may for example be from 1.0 mM to 5.0 M, preferably from 5.0 mM to 1.0 M, more preferably from 10 mM to 200 mM, most preferably from 25 to 100 mM. The medium of the fluid and the conditions thereof can be appropriately chosen depending on the components of the fluid to be separated (contaminants and target components).

In the first step of the method of the present invention, the filter system according to the present invention as described above is provided. The first synthetic non-woven layer is defined as the upstream side of the filter system and the membrane layer is defined as the downstream side of the filter system.

In the second step, the fluid to be clarified is passed through the filter system from the upstream side to the downstream side of the filter system to retain the cells and other contaminants in the filter system while eluting the fluid containing the one or more target components from the filter system.

In an optional third step, the fluid containing the one or more target components eluted from the filter system is passed in-line through a further purification device such as e.g. a membrane adsorber (for example cellulose-based or agarose-based), monoliths, resin materials, etc. to further purify the one or more target component(s). Particularly preferred are anionic exchangers, cationic exchangers, hydrophobic interaction or mixed mode chromatography. Due to the excellent filtration performance of the filter system of the present invention, it is advantageously possible to use the filter system in-line as a depth filter/clarification filter prior to a chromatography step in a downstream purification process after cell cultivation.

The filter system of the present invention advantageously shows an excellent performance in removing unwanted particles in a cell culture such as e.g. cells, lipids and large particulates from a cell culture sample containing a target component such as e.g. an antibody or a virus particle, and also obtains a high product yield at the same time. The filter system has improved filterability (up to 40 million cells/mL) and releases less TOC when compared to conventional depth filters known in the art, and can be used dry, i.e. with no venting and pre-flushing required. Since the filter system does not need pre-venting and large amounts of flushing prior to and after filtration and has a very low void volume, the target component in the filtrate has an advantageously high concentration so that further analytical measurements or processes can be performed without an additional concentration step. The turbidity of a fluid to be clarified can advantageously be reduced to a high degree (>90% or < 50 to 100 NTU) by the filter system, and the filter system of the present invention can be used as a depth filter/clarification filter prior to a chromatography step in a downstream purification process after cell cultivation. Due to the excellent performance of the filter system of the present invention in throughput (filtration capacity such as > 1mL/cm²), it is advantageously possible to directly (in-line) load the eluted fluid from the filter system to a chromatography material, and no by-pass of the chromatography material is needed during pre-flushing.
Figure 1 shows the test setup for Examples 1 to 6.
Figure 2 shows the filtration results obtained in Example 1.
Figure 3 shows the comparison of the final membrane layer of Example 3 with respect to filtrate volume and turbidity reduction.
Figure 4 shows the test results of Example 4.
Figure 5 shows the filtration results of Example 5.
Figure 6 shows the product yield of a 20 cm² filter system after rinsing the filter with buffer after filtration in Example 6.
Figure 7 shows the TOC release results of Example 7.

The present invention will be further illustrated in the following examples without being limited thereto.

### Examples

### Test setup for the following Examples 1 to 6:

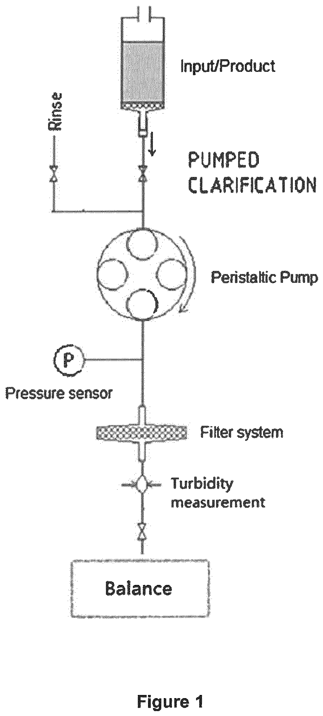

In all cell culture filtration applications, forward flow filtration was applied with constant flow rate at 10 mL/min up to 1.0 bar inlet pressure using a peristaltic pump and pressure sensor. In Example 4 and 5, filtration was performed up to 2 bar inlet pressure. The Experiments were performed using the test set up depicted in Figure 1. The product was filtered using a peristaltic pump up to 1 bar inlet pressure (Examples 1, 2, 3 and 6. The filtrate was collected in the output side of the filter system. Turbidity was measured using a turbidity meter to define particle reduction in the filtrate. In addition, filtrate weight was measured using a lab balance to determine the product weight and filter capacity.

The following non-woven layers and membrane layers were used:

| Non-woven | Material | Avr. Thickness [µm]* | Avr. Pore size [µm]** |
|---|---|---|---|
| NW1 | PP - Meltblown | 145 | 25 |
| NW2 | PP - Meltblown | 145 | 20 |
| NW3 | PP - Meltblown | 297 | 12 |
| NW4 | PP - Meltblown | 158 | 6 |
| NW5 | PET | 200 | 25 |
| NW6 | PP - Meltblown | 235 | 2.5 |
| NW7 | PP - Meltblown | 185 | 1.6 |

| | | | |
|---|---|---|---|
| * Average thickness measured according to DIN EN ISO 12625-3 using Universal thickness gauge S16502 (Frank-PTI). ** Average pore size measured according to ASTM E 1294-89 (1999) using Topas PSM 165 (porometer). | | | |

| Membrane | Material | Structure | Avr. Pore size [µm]** |
|---|---|---|---|
| Mem1 | RC | asymmetrical | 0.41 |
| Mem2 | RC | symmetrical | 0.68 |
| Mem3 | RC | symmetrical | 0.46 |
| Mem4 | CA | symmetrical | 1.0 |
| Mem5 | CA | symmetrical | 0.40 |
| Mem6 | CA | symmetrical | 1.2 |
| Mem7 | CA | symmetrical | 0.63 |
| Mem8 | CA | symmetrical | 2.1 |

| | | | |
|---|---|---|---|
| RC... regenerated cellulose CA... cellulose acetate ** determined as described hereinabove | | | |

### Example 1:

Performance trials with real solutions commercially available from several distributers were performed.

The following filter systems were used:
DF-A: NW1 + NW2 + NW3 + Mem1
DF-B: NW1 + NW2 + NW5 + Mem1
DF-C: NW1 + NW2 + NW3 + NW4 + Mem1
DF-D: NW1 + NW2 + NW3 + NW6 + Mem1
DF-E: NW1 + NW2 + NW3 + NW7 + Mem1
FC-PES: commercially available filter system based on polyethersulfone (Whatman GD/X Syringe Filters - Prefilter, Sterile | Cytiva )

DF-C is a filter system according to the present invention. DF-A and DF-B are comparative filter systems since no fourth synthetic non-woven layer is present. DF-D and DF-E are comparative filter systems since the average pore size of the fourth synthetic non-woven layer is too small. FC-PES is a comparative filter system which is commercially available.
Sample 1 cell culture information:
   Test A: CHO K1A cell line, 72% viability, 12 Mio/mL VCC, 490 NTU turbidity
   Test B: CHO K1A cell line, 50% viability, 9 Mio/mL VCC, 370 NTU turbidity
Sample 2 cell culture Information:
   Test C: ExpiCHO transient cell line 85% viability, VCC 16 Mio/mL
   Test D: HEK293 transient cell line 61% viability, VCC 9 Mio/mL
   Test E: CHO stable cell line 94% viability, VCC 18 Mio/mL

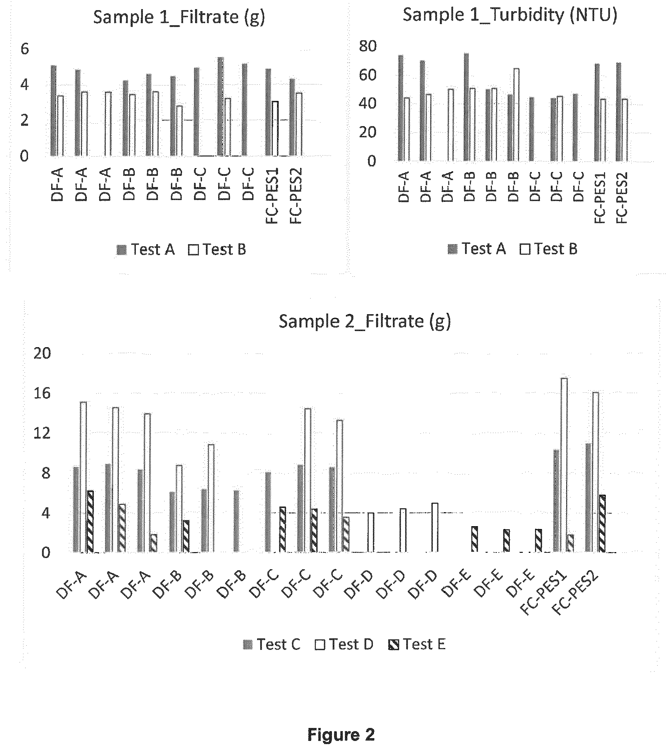

Figure 2 shows the filtration results for Samples 1 and 2 using different cell lines. As evident from Figure 2, the filter system of the present invention (DF-C) shows the best performance with different cell cultures when taking both of product yield and turbidity reduction into account.

### Example 2:

Three filtration systems (each consisting of NW1+NW2+NW3+NW4 combined with a different membrane layer) according to the present invention and a comparative filtration system consisting only of NW1+NW2+NW3+NW4 were evaluated in their performance regarding turbidity reduction and filter capacity.

The results from filtration of model solution of 10xCaro/Ovo + 20 g/L dry Yeast mixture are shown in the following Table. Turbidity of the feed solution was around 600 NTU.

**Table 1:**

| Membrane(s) | Material | Turbidity [NTU] | Filter capacity [mL/cm²] |
|---|---|---|---|
| Mem2 + Mem3 | 2 × RC | 10.6 | 1.70 |
| Mem1 | RC | 9.2 | 2.00 |
| Mem4 + Mem5 | 2 × CA | 9 | 1.70 |
| --- | | 56.40 | 2.10 |

The filter systems of the present invention show a high turbidity reduction and a similar filter capacity when compared to a filter system not comprising a final membrane layer.

### Example 3:

Five filtration systems (each consisting of NW1+NW2+NW3+NW4 combined with a different membrane layer) according to the present invention were evaluated in their performance regarding turbidity reduction and filter capacity.

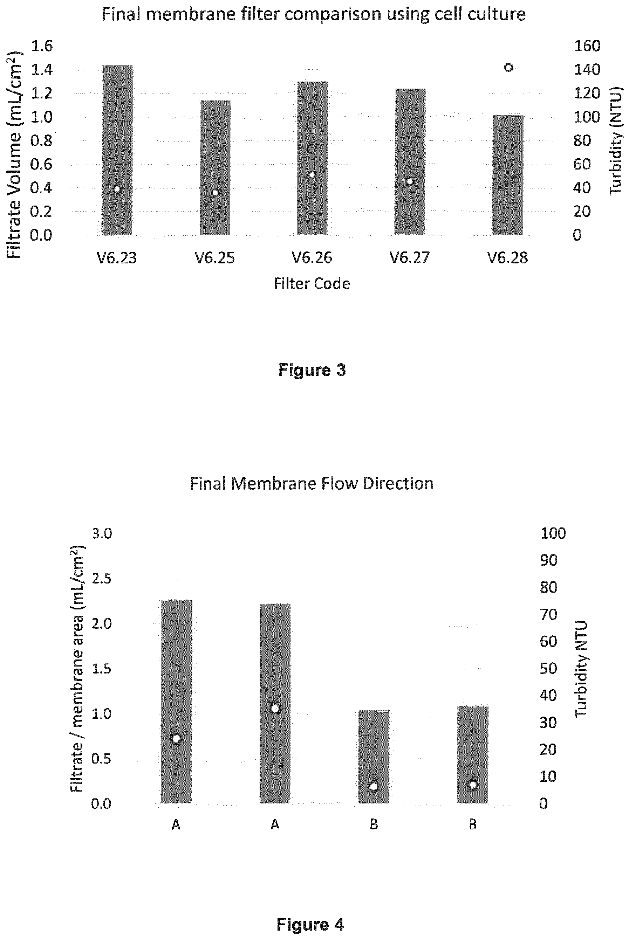

The following Table and Figure 3 show the tested filtration systems and the realized filtrate volume and turbidity. The test medium was average 12 Mio/mL CHO Cell Line.

| Filter Code | Membrane layers | Average pore size of membrane layers [µm] |
|---|---|---|
| V6.23 | Mem1 | 0.41 |
| V6.25 | Mem4 + Mem5 | 1.0 + 0.40 |
| V6.26 | Mem6 + Mem7 | 1.2 + 0.63 |
| V6.27 | Mem2 + Mem3 | 0.68 + 0.46 |
| V6.28 | Mem8 + Mem6 | 2.1 + 1.2 |

### Example 4:

The final membrane layer (Mem1; asymmetrical membrane layer, in which the first main surface has an average pore size larger than the average pore size of the second main surface) was tested regarding its orientation (A: first main surface adjacent to the fourth synthetic non-woven layer; B: second main surface adjacent to the fourth synthetic non-woven layer). The setup was NW1 + NW2 + NW3 + NW4 + Mem1. The test medium was a model solution of 10xCaro/Ovo + 20 g/L dry Yeast mixture, turbidity around 3,000 NTU. The results are shown in Figure 4.

As evident from Figure 4, orientation A (i.e. first main surface having an average pore size larger than the average pore size of the second main surface of the final membrane layer is adjacent to the fourth synthetic non-woven layer) performed best regarding filtrate volume.

### Example 5:

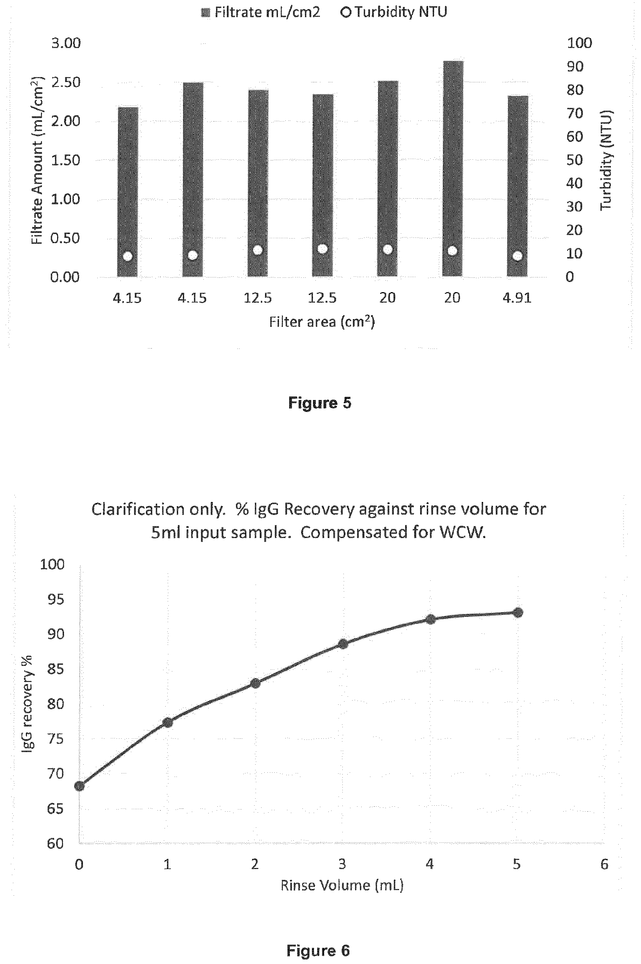

Scalability of final membrane layer combination was tested. The setup was NW1 + NW2 + NW3 + NW4 + Mem1. Figure 4 shows the normalized filtrate amount and turbidity of different filter areas. The medium was a model solution of 10xCaro/Ovo + 20 g/L dry Yeast, turbidity 4,500 NTU. The results are shown in Figure 5.

As evident from Figure 5, also the 20 cm² module was performing well and allowed a filtration volume of >50 mL per module. Accordingly, scalability of the filter system of the present invention is possible.

### Example 6:

The amount of flushing/rinsing volume needed to recover a target component from the filter system of the present invention was tested. The setup was NW1 + NW2 + NW3 + NW4 + Mem1.

In particular, Figure 6 shows the IgG yield/recovery after flushing. The cell culture was CHO cell line, 82% viability, IgG titer 2.3 g/L, WCW 53 g/L. The flushing buffer was PBS (phosphate buffered saline) at a pH of 7.

A flushing/rinsing volume of 2 mL was sufficient to achieve 80% yield/recovery. Thus, only a low amount of flushing/rinsing volume is needed to recover a target component from the filter system of the present invention.

### Example 7:

A filtration system according to the present invention (Filter system 3; Setup: NW1 + NW2 + NW3 + NW4 + Mem1) and three comparative filtration systems (Filter System 1: NW1 + NW2 + NW3 + Mem1; Filter system 2: NW5 + NW1 + NW3 + glass fiber non-woven layer + Mem1; Filter system 4: FC-PES) were evaluated in their TOC release.

TOC of clarification filters was determined by flushing different fraction of purified water. Each fraction after flushing was collected and TOC was determined using TOC LCPH system according SOP: PD/SOP/TOC/111. Steps are given below
- Flush the test setup for 2-3 hours using Arium water
- Collect the Arium water through the setup - taken as Blank
- Connect the prototype - inlet facing upward
- Maintain constant flowrate of 10 mL/min & collect each 5 mL fraction until 50 mL (0-5, 5-10....45-50 mL) dilute 1:3 dilution to each sample until 50 mL and measure TOC.
- Collect each 10 mL after 50 mL until 100 mL (50-60, 60-70...90-100 mL) make 1:2 dilution for sample from 50-100 mL and measure TOC.

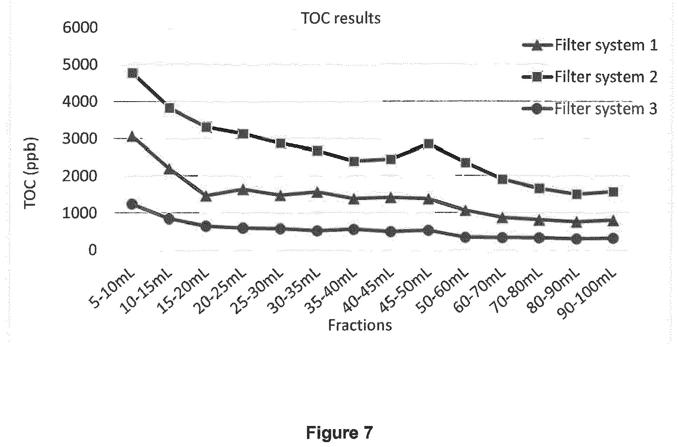

The results are shown in Figure 7.

As evident from Figure 7, a filter system comprising a glass fiber non-woven layer releases the most TOC.

### Example 8:

Various synthetic non-woven layers and Mem1 were evaluated individually with respect to their TOC release. That is, 50 mm filter layers were tested for TOC by shaking the layers in 100 mL purified water at 100 rpm for 2 hours.

The results are shown in the following Table.

| Layer | TOC (ppb) after shaking at 100rpm |
|---|---|
| Glass fiber non-woven | Filter was dissolved |
| NW1 | 1073.5 |
| NW2 | 1478.5 |
| NW3 | 1145.5 |
| NW4 | 531.6 |
| Mem1 | 2557 |

As evident from the above Table, the glass fiber non-woven layer was not resistant against water, whereas NW1 to NW4 and Mem1 only released small amount of TOC.

## Claims

1. A filter system comprising, in this order,
a first synthetic non-woven layer having a first average pore size A;
a second synthetic non-woven layer having a second average pore size B;
a third synthetic non-woven layer having a third average pore size C;
a fourth synthetic non-woven layer having a fourth average pore size D; and
a membrane layer having a fifth average pore size E,
wherein
the first average pore size A is from 15.0 µm to 50.0 µm,
the second average pore size B is from 7.50 µm to 30.0 µm,
the third average pore size C is from 5.00 µm to 15.0 µm,
the fourth average pore size D is from 3.00 µm to 10.0 µm,
the fifth average pore size E is from 0.010 µm to 2.5 µm, and
A>B>C>D>E.

2. The filter system according to claim 1, wherein the first, second, third and fourth synthetic non-woven layers are each composed of meltblown polypropylene.

3. The filter system according to claim 1 or 2, wherein the first, second, third and fourth synthetic non-woven layers each have an average thickness of from 20 to 20,000 µm.

4. The filter system according to any one of claims 1 to 3, wherein the membrane layer is a hydrophilic membrane layer having a water contact angle of 90° or less.

5. The filter system according to any one of claims 1 to 4, wherein the membrane layer has a first main surface adjacent to the fourth synthetic non-woven layer and an opposite second main surface, and the average pore size at the first main surface is larger than the average pore size at the second main surface.

6. The filter system according to any one of claims 1 to 5, wherein the membrane layer is an asymmetrical membrane layer.

7. The filter system according to any one of claims 1 to 6, wherein the membrane layer is a single membrane layer.

8. The filter system according to any one of claims 1 to 7, wherein the membrane layer is composed of one or more polymers selected from the group consisting of cellulose and derivatives thereof, regenerated cellulose, polyamides, fluoropolymers, polyolefins, polysulfons, poly(meth)acrylic acid, and acrylic acid/methacrylic acid copolymers.

9. The filter system according to any one of claims 1 to 8, wherein the membrane layer is composed of regenerated cellulose or cellulose acetate.

10. The filter system according to any one of claims 1 to 9, wherein the fifth average pore size E is from 0.20 µm to 0.70 µm.

11. The filter system according to any one of claims 1 to 10, wherein the membrane layer has a thickness of from 20 to 400 µm.

12. A method of separating cells and other contaminants from a fluid containing one or more target components, the method comprising
providing the filter system according to any one of claims 1 to 11, wherein the first synthetic non-woven layer is defined as the upstream side of the filter system and the membrane layer is defined as the downstream side of the filter system, and passing the fluid through the filter system from the upstream side to the downstream side of the filter system to retain the cells and other contaminants in the filter system while eluting the fluid containing the one or more target components from the filter system.

13. The method according to claim 12, further comprising passing the fluid containing the one or more target components eluted from the filter system in-line through a purification device to further purify the one or more target components.
